# EUROPEAN PATENT APPLICATION

(11) **EP 1 994 944 A1**
(43) Date of publication of application: **26.11.2008**
(21) Application number: 07010028.4
(22) Date of filing: 21.05.2007
(51) Int. Cl.: A61K 47/48

(54) **Polymer conjugate compounds for the inhibition of apoptosis**

(71) Applicant: Laboratorios SALVAT, S.A., 08950 Esplugues de Llobregat, Barcelona (ES); CONSEJO SUPERIOR DE INVESTIGACIONES CIENTIFICAS, 28006 Madrid (ES)
(72) Inventor: Messeguer Peypoch, Angel, 08025 Barcelona (ES); Moure Fernandez, Alejandra, 08940 Cornelia de Llobregat-Barcelona (ES); Sanclimens Perez de Rozas, Gloria, 08011 Barcelona (ES); Perez-Paya, Enrique, 46020 Valencia (ES); Mondragon Martinez, Laura, 12480 Soneja-Castellon (ES); Vicent Docon, Maria Jesus, 12540 Vila-Real, Castellon (ES); Orzaez Calatayud, Mar, 46006 Valencia (ES); Malet Engra, Gema, 46920 Mislata-Valencia (ES)
(74) Representative: Carvajal y Urquijo, Isabel

(57) **Abstract**

The present invention relates to polymer conjugate compounds, as well as to processes for their preparation, to pharmaceutical compositions containing them and their use in medicine.

## Description

The present invention relates to polymer conjugate compounds, as well as to processes for their preparation, to pharmaceutical compositions containing them and their use in medicine.

### BACKGROUND ART

To accomplish the full therapeutic potential of a bioactive agent it is crucial to localize it to the disease location and once there, to ensure in time its efficient release and availability to the required cellular compartment.

The original idea of covalently conjugating a low molecular weight drug to a hydrophilic polymeric carrier to increase its therapeutic effect was proposed by Helmut Ringsdorf in 1975 (Ringsdorf, H. 1975, J. Polym. Sci. Pol. Symp., 51, 35-53). Ringsdorf's model consisted of four different components: a polymeric carrier, a drug, a biodegradable linker and a targeting group. It was envisaged that covalent conjugation of a low molecular weight drug would alter drug pharmacokinetics at cellular level by restricting its uptake to the endocytic route. In addition, the hydrophilic polymeric backbone would also increase drug solubility as conventional agents are often hydrophobic and therefore poorly soluble in biological fluids. These conjugates were designed to obtain selective drug release in the lysosomal compartment ("lysosomotropic delivery") (De Duve et al. 1974, Biochem. Pharmacol., 23, 2495-531). As a consequence, the linker emerged immediately as a key feature. Whilst stable in the blood stream, it needs to be degraded by a biological trigger of lysosomes (usually low pH or enzymes, in particular thiol proteases) due to the accumulation of conjugates in this intracellular compartment following endocytic capture. In the 80s and early 90s a vast number of preclinical studies were carried out to optimise the characteristics of the polymeric carrier, of the linker (Duncan et al. 1980, Biochem. Bioph. Res. Co., 94, 284-290; Duncan et al. 1981, Biochem. Biophys Acta, 678, 143-150; Duncan et al. 1983, Makromol. Chem., 84, 1997-2008) and to prove the safety of these novel constructs (Duncan et al. 1992, J. Control. Release, 19, 331-346).

Cathepsin B has well-established endopeptidase activity but also exhibits an important carboxyldipeptidyl activity (Cezari et al. 2002, Biochem J., 368, 365-369; Kirschke et al. Lysosomal Cysteine Proteases. 1998. Oxford University Press; Portaro et al. 2000, Biochem J., 347, 123-129). However, within polymer conjugates field the endopeptidase activity is the one of interest. Linkers described in the prior art as appropriate for cathepsin B cleavage involve a Gly residue at P1 position (ie, the position covalently bound to the bioactive agent) (Duncan et al. 1980, Biochem. Bioph. Res. Co., 94, 284-290; Duncan et al. 1981, Biochem. Biophys Acta, 678, 143-150; Duncan et al. 1983, Makromol. Chem., 84, 1997-2008).

Recently, the first anti-apoptotic polymer drug conjugates have been described (Vicent, MJ & Pérez-Payá E, 2006, J. Med. Chem. 49, 3763-3765; Malet et al. 2006 Cell Death Differ. 13, 1523-1532). These conjugates employ a diglycil sequence as a linker.

However, experimental results have shown that for some applications the drug release rate for compounds comprising a Gly residue at P1 is too slow to achieve the effective concentrations in the desired time range and therefore there is the need of the provision of linker sequences which permit to obtain drug release rates within the suitable time interval.

A deeper study of these systems regarding the polymer-drug linker has revealed a good but unexpected improvement. By changing Gly at P1 position by an amino acid selected from Val, Leu, Ile, Ala, Arg and Phe, it is possible to markedly modulate drug release rate. This could be translated in the possibility to use the same biological agent in different human pathologies where a specific and fast drug release is required such as acute lesions or organ transplantation.

### DESCRIPTION OF THE INVENTION

One aspect of the present invention relates to polymer conjugate compounds of general formula **I,** 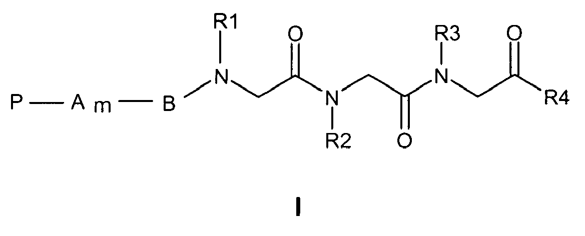 wherein:
R1, R2 and R3 independently represent -(CH₂)₀₋₃-(3-6)Cy, -(CH₂)₀₋₃-(5-6)Hetcy, -(CH₂)₁₋₃-phenyl, -(CH₂)₁₋₃-(1-naphthyl), -(CH₂)₁₋₃-(2-naphthyl), - (CH₂)₁₋₃-(5-10)Hetar, -(CH₂)₂-CH(phenyl)₂, -(CH₂)₂-CH(phenyl)[(5-6)Hetar] or -(CH₂)₂-CH[(5-6)Hetar]₂;
R4 represents -OR5 or -NR5R6
R5 and R6 independently represent -H or -(C₁-C₃)alkyl
(3-6)Cy represents a radical of a 3- to 6-membered partially unsaturated or saturated carbocyclic ring;
(5-6)Hetcy represents a C- or N- radical of a 5- or 6-membered, partially unsaturated or saturated carbocyclic ring containing from one to two heteroatoms independently selected from O, S and N;
Cy and Hetcy can be optionally substituted with one or more substituents selected from halogen, -CF₃ and -OH;
(5-6)Hetar and (5-10)Hetar represent a C- or N- radical of an aromatic 5-or 6-membered and 5- to 10-membered ring respectively; containing from one to four heteroatoms independently selected from O, S and N;
phenyl, 1-naphthyl, 2-naphthyl, (5-6)Hetar and (5-10)Hetar may be optionally substituted with one or more substituents selected from halogen, -CF₃, -OH, -O-(C₁-C₃)alkyl, -CO-(C₁-C₃)alkyl, -(C₁-C₅)alkyl-NR5R6, -NR5R6 and -SO₂NH₂;
B represents an amino acid selected from Val, Leu, Ile, Ala, Arg and Phe;
A independently represents any of the 20 naturally occurring amino acids, and m is 1, 2 or 3, and
P represents a linear or branched polymer selected from polyglutamic acid, polyethylene glycol, polylactic acid, polyglycolic acid, polylactic-coglycolic acid and glycopolymers,

its stereoisomers and mixtures thereof, its polymorphs and mixtures thereof and the pharmaceutically acceptable solvates and addition salts thereof.

Thus, polymer conjugate compounds of formula **I** comprise different moieties: a bioactive agent bound to a peptidic linker bound to a polymer, wherein the amino acid of the peptidic linker covalently bound to the bioactive agent is selected from Val, Leu, Ile, Ala, Arg and Phe.

In the previous definitions, the terms (C₁-C₃)alkyl and (C₁-C₅)alkyl represent straight or branched saturated hydrocarbon chains containing from one to three and from one to five carbon atoms respectively. Examples of (C₁-C₃)alkyl include, but are not limited to, methyl, ethyl, propyl, isopropyl. Examples of (C₁-C₅)alkyl include additionally and without limitation butyl, isobutyl, sec-butyl, tert-butyl, 1-pentyl, 1-methylbutyl, 2-methylbutyl, 3-methylbutyl, 1,1-dimethylpropyl, 1,2-dimethylpropyl and 2,2-dimethylpropyl.

The groups (C₁-C₃)alkyl and (C₁-C₅)alkyl may be optionally substituted according to the description whenever appropriate from a chemical point of view.

The term (3-6)Cy represents a radical of a 3- to 6-membered partially unsaturated or saturated carbocyclic ring. As mentioned previously, it can be optionally substituted with one or more substituents, which can be placed at any available position of the ring. Examples of (3-6)Cy include, without limitation, radicals of cyclopropane, cyclobutane, cyclopentane, cyclohexane, 1-cyclobutene, 2-cyclobutene, 1-cyclopentene, 2-cyclopentene, 1-cyclohexene, 2-cyclohexene and 3-cyclohexene.

The term (5-6)Hetcy represents a C- or N- radical of a partially unsaturated or saturated 5- to 6-membered carbocyclic ring containing one or two heteroatoms independently selected from O, S and N, that may be substituted according to the description at any available ring position. Examples of (5-6)Hetcy include, without limitation, radicals of dihydrofuran, pyrroline, pyrazoline, imidazolidine, isothiazolidine, isoxazolidine, oxazolidine, pyrazolidine, pyrrolidine, thiazolidine, dioxane, morpholine, piperazine, piperidine, pyran, tetrahydropiran, oxazine, oxazoline, pyrroline, thiazoline, pyrazoline, imidazoline, isoxazoline, and isothiazoline.

The terms (5-6)Hetar and (5-10)Hetar represent a C- or N- radical of an aromatic 5- or 6-membered and 5- to 10-membered ring respectively; containing from one to four heteroatoms independently selected from O, S and N, that may be substituted according to the description at any available ring position. Examples of (5-6)Hetar include, without limitation, radicals of pyrrol, furan, tiophene, imidazole, isoxazole, isothiazole, oxazole, 1,2,4-oxadiazole, 1,2,4-thiadiazole, 1,3,4-oxadiazole, 1,3,4-thiadiazole, 1,2,3-triazole, 1,2,4-triazole, pyridine, pyrimidine, pyridazine and pyrazine. Examples of (5-10)Hetar further include, without limitation, radicals of benzimidazole, benzofuran, benzothiazole, benzothiophene, imidazopyrazine, imidazopyridazine, imidazopyridine, imidazopyrimidine, indazole, indole, isoindole, isoquinoline, pyrazolopyrazine, pyrazolopyridine, pyrazolopyrimidine, purine, quinazoline, quinoline and quinoxaline.

The expression "optionally substituted with one or more" means that a group can be either unsubstituted or substituted with one or more, preferably with 1, 2, 3 or 4 substituents, provided that this group has 1, 2, 3 or 4 positions susceptible of being substituted.

Throughout the description and claims the word "comprise" and variations of the word, such as "comprising", are not intended to exclude other additives, components, elements or steps.

As used therein the term "treatment" includes treatment, prevention and management of such condition. The term "pharmaceutically acceptable" as used herein refers to those compounds, compositions, and/or dosage forms which are, within the scope of medical judgement, suitable for use in contact with the tissues of humans and animals without excessive toxicity, irritation, allergic response, or other problem or complication, commensurate with a reasonable benefit/risk ratio.

In a particular embodiment of the invention R1 represents -(CH₂)₂-phenyl optionally substituted with one or more substituents selected from halogen. In another embodiment R1 represents 2,4-dichlorophenylethyl.

In another embodiment R2 represents -(CH₂)₁₋₃-aryl optionally substituted, or - (CH₂)₂-CH(Ph)₂ optionally substituted. In another embodiment R2 represents 3,3-diphenylpropyl.

In another embodiment R3 represents -(CH₂)₂-phenyl optionally substituted with one or more substituents selected from halogen. In another embodiment R3 represents 2,4-dichlorophenylethyl.

In another embodiment R2 represents 3,3-diphenylpropyl and R1 and R3 both represent 2,4-dichlorophenylethyl.

In another embodiment of the invention R4 represents -NR5R6 and R5 and R6 both represent -H.

In another embodiment of the invention B represents Val.

In another embodiment of the invention B represents Leu.

In another embodiment of the invention B represents Ile.

In another embodiment of the invention B represents Ala.

In another embodiment of the invention B represents Arg.

In another embodiment of the invention B represents Phe.

In the polymer drug conjugate, the polyglutamic acid may be poly(L-glutamic acid), poly(D-glutamic acid) or poly(DL-glutamic acid). In one embodiment of the invention polyglutamic acid is poly(L-glutamic acid).

In a particular embodiment of the invention, R1 and R3 both represent 2,4-dichlorophenylethyl, R2 represents 3,3-diphenylpropyl, R4 represents - NR5R6, R5 and R6 both represent -H, B represents Val and P represents polyglutamic acid.

Furthermore, all possible combinations of the above-mentioned embodiments form also part of this invention.

The compounds of the present invention may contain one or more basic nitrogen atoms and, therefore, they may form salts with acids, that also form part of this invention. Examples of pharmaceutically acceptable salts include, among others, addition salts with inorganic acids such as hydrochloric, hydrobromic, hydroiodic, nitric, perchloric, sulphuric and phosphoric acid, as well as addition salts of organic acids such as acetic, methanesulfonic, trifluoromethanesulfonic, ethanesulfonic, benzenesulfonic, p-toluenesulfonic, benzoic, camphorsulfonic, mandelic, oxalic, succinic, fumaric, tartaric, and maleic acid. Likewise, compounds of the present invention may contain one or more acid protons and, therefore, they may form salts with bases, that also form part of this invention. Examples of these salts include salts with metal cations, such as for example an alkaline metal ion, an alkaline-earth metal ion or an aluminium ion; or it may be coordinated with an organic with an organic or inorganic base. An acceptable organic base includes among others diethylamine and triethylamine. An acceptable inorganic base includes aluminium hydroxide, calcium hydroxide, potassium hydroxide, sodium carbonate, and sodium hydroxide. There may be more than one cation or anion depending on the number of functions with charge and on the valency of cations and anions.

Salts derived from pharmaceutically acceptable organic nontoxic bases include salts of primary, secondary, and tertiary amines, substituted amines including naturally occurring substituted amines, cyclic amines, and basic ion exchange resins, such as arginine, betaine, caffeine, choline, N,N-dibenzylethylenediamine, diethylamine, 2-diethylaminoethanol, 2-dimethylaminoethanol, ethanolamine, ethylenediamine, N-ethylmorpholine, ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine, tromethamine, and the like.

There is no limitation on the type of salt that can be used, provided that these are pharmaceutically acceptable when they are used for therapeutic purposes. Salts can be synthesized from the parent compound which contains a basic or acidic moiety by conventional chemical methods. Generally, such salts can be prepared by reacting the free acid or base forms of these compounds with a stoichiometric amount of the appropriate base or acid in water or in an organic solvent, such as ether, ethyl acetate, ethanol, isopropanol, or acetonitrile or in a mixture of both. The compounds of formula I and their salts differ in some physical properties but they are equivalent for the purposes of the present invention.

Some of the compounds of formula I of the present invention may exist in unsolvated as well as solvated forms such as, for example, hydrates. The present invention encompasses all such above-mentioned forms which are pharmaceutically active.

Some of the compounds of general formula I may exhibit polymorphism, encompassing the present invention all the possible polymorphic forms, and mixtures thereof. Various polymorphs may be prepared by crystallization under different conditions or by heating or melting the compound followed by gradual or fast cooling. The presence of polymorphs may be determined by solid NMR spectroscopy, IR spectroscopy, differential scanning calorimetry, powder X-ray diffraction or such other techniques.

Another aspect of the present invention relates to a process for the preparation of polymer conjugate compounds of formula I, their derivatives, their analogues, their tautomeric forms, their stereoisomers, their polymorphs or their pharmaceutical acceptable salts and solvates.

The compounds of the present invention may be synthesized using the methods described below, as well as by other processes known in the field of organic synthesis. Preferred methods include, but are not limited to, the general processes shown in the attached schemes. Unless otherwise stated the groups R1, R2, R3, R4, m, x and y have the meaning described in general formulas **I** and **II.**

Compounds of formula **II** can be obtained using the following method: 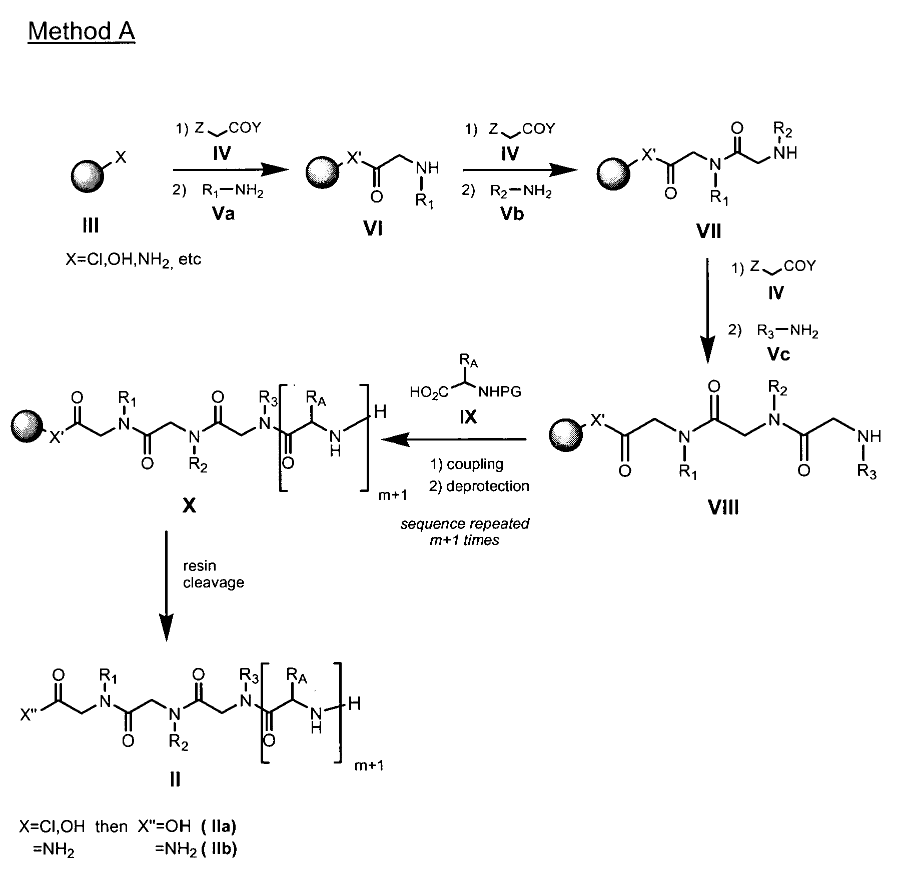 RA represents the side chain of the corresponding amino acid in each position.

According to Method A, when X is -NH2, as for example Rink amide resin, upon deprotection from fluorenylmethoxycarbonyl group, the solid support amine **III** is acylated with an acylating agent **IV,** wherein X represents a leaving group, for example halogen and Y represents -OH or halogen. When Y represents halogen, for example chloroacetylchloride the reaction can be carried out in the presence of a tertiary base as triethylamine. When Y represents -OH, for example bromoacetic acid the reaction can be carried out in the presence of a suitable coupling agent, for example *N,N'-*diisopropylcarbodiimide. In both cases, reaction can be performed in an inert solvent capable of swelling correctly the resin, such as *N,N'-*dimethylformamide or dichloromethane and at room temperature or using microwave irradiation, decreasing reaction time. Then, an amine Va is coupled using a tertiary amine as a base. The reaction can be carried out at room temperature, by heating, for example at 60°C, or by MW irradiation. After repetition of acylation and amination in the same way as described for the intermediate **VI,** intermediates **VII** and **VIII** are obtained. Intermediate X can be obtained using standard reactions for the synthesis of peptides. Thus, the solid support intermediate **VIII** is firstly reacted with a protected aminoacid **(IXa)** and optionally subsequently reacted with another protected aminoacid **(IXb).** After coupling the protecting group is removed. This sequence can be repeated up to four times. Final compound **II** can be released from the resin using a suitable removing agent as for example a mixture of trifluoroacetic acid in dichloromethane.

In another case, when the final compound **II** is a carboxylic acid, resins like Merrifield, chlorotrityl chloride, Rink acid, Wang or activated versions of these polymers, containing reacting halogen atoms or hydroxyl groups, can be used as **III.** Seneci, P. Solid-phase Synthesis and Combinatorial Technologies; Wiley & Sons: New York, 2000; Burgess, K., Solid-Phase Organic Synthesis; Ed.; John Wiley & Sons: New York, 2000; Zaragoza Dörwald, F. Organic Synthesis on Solid Phase, 2nd ed.; Wiley-VCH: Weinheim, Germany, 2002. When X is -Cl coupling with **IV** can be carried out in the presence of a tertiary base as diisopropylethylamine. When X is OH reactions can be achieved by DMAP catalysed esterification in the presence of a suitable coupling agent as *N*,*N*'-diisopropylcarbodiimide.

An alternative strategy for obtaining a compound of formula **II** involves acylation of **III, VI** and **VIII** with amino acids of formulas **Xla-c** using standard reactions for the synthesis of peptides. A suitable coupling agents such as *O-*(7-azabenzotriazol-1-yl)-*N,N,N',N'*-tetramethyluronium hexafluoro phosphate (HATU), diisopropylcarbodiimide (DIC) or benzotriazole-1-yloxy-tris-pyrrolidinophosphonium (PyBOP) may be used, with DCM or DMF as more appropriate solvents (Method B).

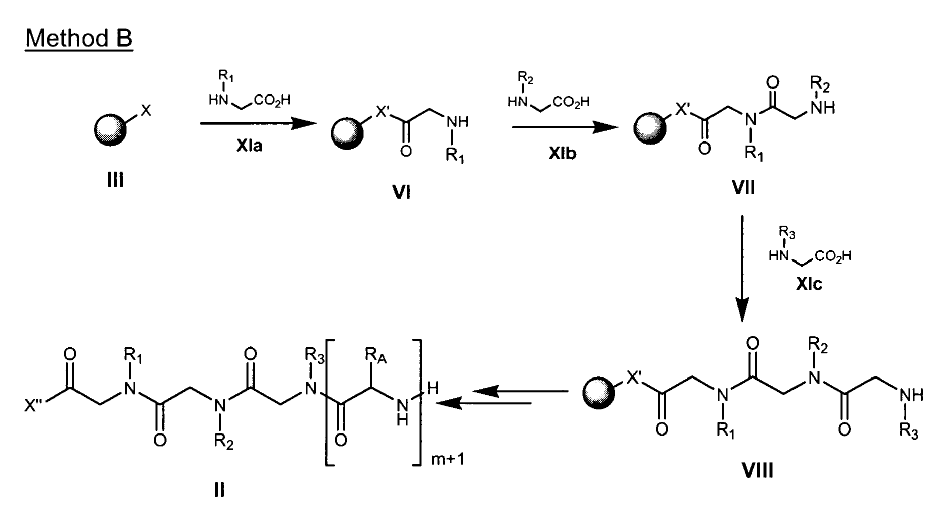

As it will be obvious to one skilled in the art, it is possible to combine some steps of method A with some steps of method B to obtain a compound of formula **II.**

Starting primary amines Va, Vb and Vc are commercially available, or may be obtained by known methods (March, Advanced Organic Chemistry, 1991, Ed. John Wiley & Sons) or using for example the scheme described below.

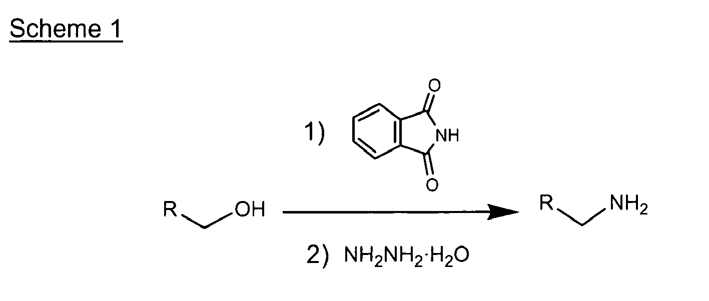

An amine can be obtained by Mitsunobu reaction starting from an alcohol and potassium phthalimide in the presence of, for example, diethyl azodicarboxylate (DEAD) and triphenylphosphine in tetrahydrofurane as a solvent and further cleavage with hydrazine hydrate (Mitsunobu, J. Am. Chem. Soc. 1972, 94, 679-680)

*N*-Substituted glycines **XIa, XIb and XIc** may be synthesized by the methods shown below either by reductive amination of the corresponding glycine with the suitable aldehyde (Scheme 2) using reducing agents such as NaBH₄, NaBH₃CN or NaBH(AcO)₃, or by nucleophilic substitution of an ester with the suitable amine E-NH2 (Scheme 3).

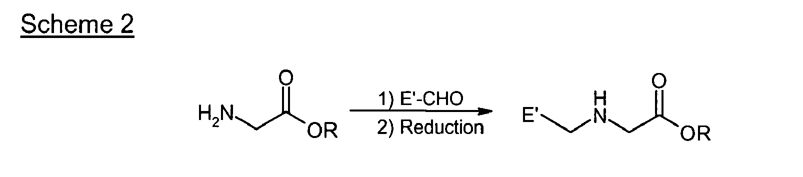

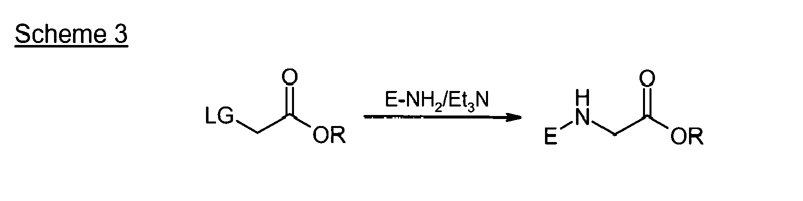

Conversion of compounds of formula **II** into different one involves transforming -COOH into a different group. The modifications considered are: the esterification of the resulting carboxylic acid **(IIc);** and, lastly, the amination to obtain the corresponding amides **(IId).** These processes are those commonly used. (Scheme 4)

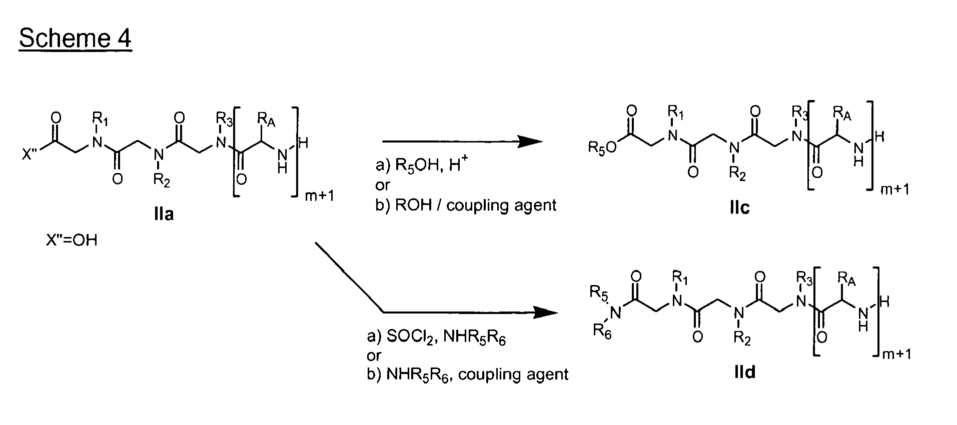

Amide coupling between **II** and polyglutamic acid **XIIa** (obtained from the corresponding salt **XII)** yields the drug conjugate **I.**

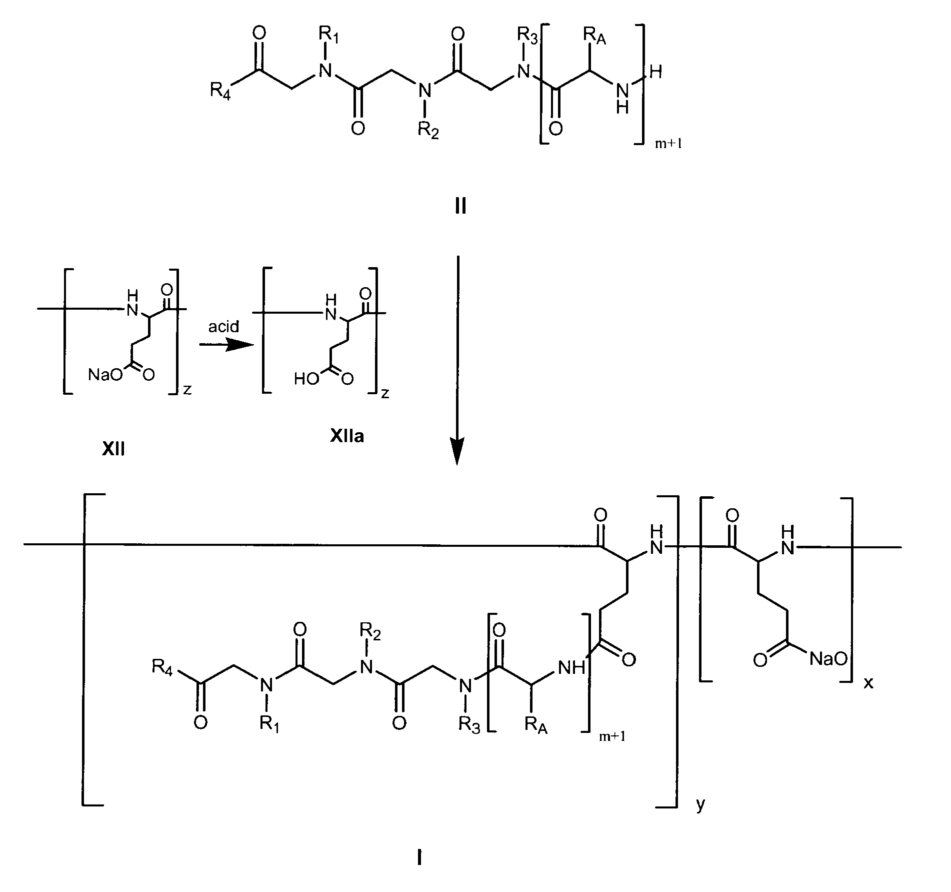

In another embodiment of the invention, the ratio of y (bioactive agent + peptidic linker) to x (polymer) is 25-30% to 65-60% by weight.

The compounds of the present invention are Apaf-1 inhibitors. Therefore, they are useful for the treatment or prevention of a condition associated with the induction of apoptosis.

Some apoptotic signals activate the mitochondria-mediated or intrinsic pathway that utilises caspase-9 as its initiator. The formation of the macromolecular complex named apoptosome is a key event in this pathway.

The apoptosome is a holoenzyme multiprotein complex formed by cytochrome c-activated Apaf-1 (apoptotic protease-activating factor), dATP and procaspase- 9 (Li et al., Cell 1997, 91: 479-489; Acehan et al., Mol. Cell 2002, 9: 423-432; Rodriguez et al., Genes Dev. 1999, 13: 3179-3184; Srinivasula et al., Mol. Cell 1998, 1: 949-957). In this macromolecular complex, apoptosome associated caspase-9 is activated and then, in turn, activates effector caspases. To identify molecules that could ameliorate disease-associated apoptosis, drug discovery efforts have initially targeted caspase activity, and thus sought specific enzymatic activators or inhibitors (Scott et al., J. Pharmacol. Exp. Ther. 2003, 304: 433-440; Garcia-Calvo M, J. Biol. Chem. 1998, 273: 32608-32613), rather than modulators in the upstream activation cascade/pathway.

In adults, post-mitotic cells, such as neurons and heart muscle cells are rarely renewed. Hence their loss can cause neurological and cardiac disorders. These cells undergo apoptotic cell death in a variety of acute and chronic degenerative diseases. Similarly, septic shock, ischaemia, and intoxication can cause massive apoptosis in multiple organs. Infections can cause an elevated apoptotic turnover of specific cell types, for instance, lymphocytes in AIDS or gastric mucosa cells in *Helicobacter pylori* infection. Apoptosis events are also of importance in procedures for ischaemic and preservation-reperfusion in organ transplantation. These diseases are therefore candidates for therapeutic cell death suppression.

Other conditions or diseases related with apoptosis include, without limitation, stroke, traumatisms, brain injury, spinal cord injury, meningitis, Alzheimer's disease, Parkinson's disease, Huntington's disease, Kennedy's disease, multiple sclerosis, prion-related diseases, as well as other acute or chronic cardiovascular diseases, alcoholic related pathologies, and treatment of allopecia.

Another aspect of the present invention relates to the use of polymer conjugate compounds of formula I for the preparation of a medicament for the treatment or prevention of a disease or condition associated with the induction of apoptosis.

Another aspect of the present invention relates to a method for the treatment or prevention of a disease or condition associated with the induction of apoptosis comprising administering a polymer conjugate compound of formula I and one or more pharmaceutical acceptable excipients.

In an embodiment of the invention, the disease or condition associated with the induction of apoptosis is stroke, traumatisms, brain injury, spinal cord injury, meningitis, Alzheimer's disease, Parkinson's disease, Huntington's disease, Kennedy's disease, multiple sclerosis, prion-related Diseases, miocardial ischaemia, as well as other acute or chronic cardiovascular diseases, organ transplantation, alcoholic related pathologies, and treatment of allopecia.

Another aspect of the present invention relates to a pharmaceutical compositions comprising a polymer conjugate compound of formula **I** or a pharmaceutically acceptable salt or solvate thereof, together with one or more pharmaceutically acceptable excipients, in either single or multiple doses. The examples of the excipients mentioned below are given by way of illustration only and are not to be construed as limiting the scope of the invention.

The compounds of the present invention can be administered in the form of any pharmaceutical formulation. The pharmaceutical formulation will depend upon the nature of the active compound and its route of administration. Any route of administration may be used, for example such as oral, buccal, pulmonary, topical, parentheral (including subcutaneous, intramuscular, and intravenous), transdermal, ocular (ophthalmic), inhalation, intranasal, otic, transmucosal, implant or rectal administration. However oral, topical or parentheral administration are preferred.

Solid compositions for oral administration include among others tablets, granulates and hard gelatin capsules, formulated both as immediate release or modified release formulations.

Alternatively, the compounds of the present invention may be incorporated into oral liquid preparations such as emulsions, solutions, dispersions, suspensions, syrups, elixirs or in the form of soft gelatin capsules. They may contain commonly-used inert diluents, such as purified water, ethanol, sorbitol, glycerol, polyethylene glycols (macrogols) and propylene glycol. Aid compositions can also contain coadjuvants such as wetting, suspending, sweetening, flavouring agents, preservatives, buffers, chelating agents and antioxidants.

Injectable preparations for parentheral administration comprise sterile solutions, suspensions or emulsions in oily or aqueous vehicles, and may contain coadjuvants, such as suspending, stabilizing, tonicity agents or dispersing agents.

The compound can also be formulated for its topical application. Formulations include creams, lotions, gels, powders, solutions, shampoo preparations, oral paste, mouth wash preparations and patches wherein the compound is dispersed or dissolved in suitable excipients.

In one embodiment of the invention the pharmaceutical composition is in the form of nanospheres, microparticles and nanoparticles.

The effective dosage of active ingredient may vary depending on the particular compound administered, the route of administration, the nature and severity of the disease to be treated, as well as the age, the general condition and body weight of the patient, among other factors. A representative example of a suitable dosage range is from about 0.001 to about 100 mg/Kg body weight per day, which can be administered as a single or divided doses. However, the dosage administered will be generally left to the discretion of the physician.

### Caspase inhibition assay

The time-dependent inhibition of caspase induced by the compounds described in this invention was analyzed. Cell lines were grown in the presence or absence of compounds (50 µM) at different times. Caspase activity was assayed using Saos-2 or U937 cultured cell lines and Ac-DEVD-afc (N-acetyl-Asp-Glu-Val-Asp-afc(7-amino-4-trifluoromethyl coumarin)) as substrate (20 µM). For Saos-2 cell line, apoptosis induction was carried out by the addition of doxycyclin 2 µM; in the case of U937, apoptosis was induced by doxorubicin 0.25 µM. Caspase activity was continuously monitored by the liberation of fluorescent afc using a Cytofluor 4000 fluorimeter (excitation 400 nm; emission 508 nm) for 2 hour. The effect of the compounds on the caspase activity was expressed as the percentage of inhibition in the fluorescence signal of treated cells vs the non-treated cells (control). As control is shown the activity of the previously described conjugate with diglycil linker (named as diglycil)

| | **Time (h)** | **% Caspase Inhibition I.1 (50 µM drug-eq.)** | **% Caspase Inhibition I.2 (50 µM drug-eq.)** | **% Caspase Inhibition diglycil (50 µM drug-eq.)** |
|---|---|---|---|---|
| **Saos-2** | **24** | 23 | 65 | 67 |
| | **48** | 65 | 100 | 72 |
| | **72** | 100 | 100 | 100 |

### EXAMPLES

The following abbreviations have been used in the examples:
DCM: dichloromethane
DIC: N,N'-diisopropylcarbodiimide
DIPEA: diisopropylethylamine
DMF: *N,N'*-dimethylformamide
eq: molar equivalent
EtOAc: ethyl acetate
Fmoc: fluorenylmethoxycarbonyl
HOBT: 1-hydroxybenzotriazole
Leucine : L
PGA: poly(L-glutamic acid)
Phenylalanine: F
rt: retention time
TBTU: *O*-(Benzotriazol-1-yl)-*N,N,N',N'*-tetramethyluronium tetrafluoroborate
TFA: trifluoroacetic acid
Valine:V

The present invention will be further illustrated by the following examples. The examples are given by way of illustration only and are not to be construed as limiting the scope of the invention.

Polystyrene AM RAM resin (0.73 mmol/g) was purchased from Rapp Polymere GmbH (Germany). Microwave-assisted reactions were performed in a CEM Discover microwave reactor using a 100 mL glass reaction vessel. Analytical RP-HPLC was performed with a Hewlett Packard Series 1100 (UV detector 1315A) modular system using a reverse-phase Kromasil 100 C₈ (15 x 0.46 cm, 5 µm) column and a X-Terra C₁₈ (15 x 0.46 cm, 5 µm). CH₃CN-H₂O Mixtures containing 0.1% TFA at 1 mL/min was used as mobile phase and monitoring was set at 220 nm. Semi-preparative RP-HPLC was performed with a Waters (Milford, MA, U.S.A.) system using a X-Terra C₁₈ (19 x 25 cm, 5 µm). ESI-HRMS were obtained with a UPLC Acquity (Waters, Mildford, USA) coupled to a mass spectrometer model LCT Premier XE (Waters) with a TOF analyzer in a positive mode using an Acquity UPLC BEH C₁₈ column (1.7 µm 2.1 x 50 mm, Waters).

The nomenclature used in this document is based on AUTONOM (Automatic Nomenclature), a Beilstein Institute computerized system for the generation of IUPAC systematic nomenclature.

### Compounds of formula II

### II.1: 2-(2-Amino-3-phenylpropionylamino)-N-{[({carbamoylmethyl-[2-(2,4-dichlorophenyl)ethyl]carbamoyl}methyl)-(3,3-diphenylpropyl)carbamoyl]-methyl}-N-[2-(2,4-dichiorophenyl)ethyl]-3-methylbutyramide

The Rink amide resin (500 mg, 0.36 mmol) was treated with 4 mL of 20% piperidine in DMF and the mixture was stirred in a microwave reactor for 2 min at 60 °C. The resin was filtered and washed with DMF (3 x 3 mL), isopropyl alcohol (3 x 3 mL) and DCM (3 x 3 mL). The resin was treated with a solution of bromoacetic acid (**IV**, 254 mg, 5 eq.) and DIC (285 µL, 5 eq.) in DMF (3 mL). The reaction mixture was stirred for 2 min at 60 °C in a microwave reactor. The resin was drained and washed with DCM (3 x 3 mL), isopropyl alcohol (3 x 3 mL) and DMF (3 x 3 mL). A solution of 2,4-dichlorophenethylamine (**Va**, 275 µL, 5 eq.) and triethylamine (255 µL, 5 eq.) in 4 mL of DMF was added to the resin and the suspension was stirred for 2 min at 90 °C under microwave activation. The supernatant was removed and the residue was drained and washed with DMF (3 x 3 mL), isopropyl alcohol (3 x 3 mL) and DCM (3 x 3 mL). Then, the resin was treated with a solution of bromoacetic acid **(IV,** 254 mg, 5 eq.) and DIC (285 µL, 5 eq.) under the same conditions as described above. The resin was drained and washed with DCM (3 x 3 mL), isopropyl alcohol (3 x 3 mL) and DMF (3 x 3 mL). ). A solution of 3,3-diphenylpropylamine (**Vb**, 385 mg, 5 eq.) and triethylamine (255 µL, 5 eq.) in 4 mL of DMF was added to the resin and the suspension was stirred for 2 min at 90 °C under microwave activation. The supernatant was removed and the residue was drained and washed with DMF (3 x 3 mL), isopropyl alcohol (3 x 3 mL) and DCM (3 x 3 mL). Afterward, the resin was treated again with a solution of bromoacetic acid (**IV,** 254 mg, 5 eq.) and DIC (285 µL, 5 eq.) under the described conditions previously. A solution of 2,4-dichlorophenethylamine (**Vc**, 275 µL, 5 eq.) and triethylamine (255 µL, 5 eq.) in 4 mL of DMF was added to the resin and the suspension was stirred for 2 min at 90 °C under microwave activation. The supernatant was removed and the residue was drained and washed with DMF (3 x 3 mL), isopropyl alcohol (3 x 3 mL) and DCM (3 x 3 mL). An amino acid, *N*-α-Fmoc-L-Valine (**IXa**, 310 mg, 2.5 eq.), was linked to the resin using TBTU (293 mg, 2.5 eq.), HOBT (124 mg, 2.5 eq.) and DIPEA (160 µL, 2.5 eq.). The mixture was stirred for 1 h at room temperature. The resin was drained and washed with DMF (3 x 3 mL), isopropyl alcohol (3 x 3 mL) and DCM (3 x 3 mL). If necessary, the amino acid was recoupled under the same conditions. Upon removal of the Fmoc group with 4 mL of 20% piperidine in DMF (30 min), the resin was washed with DMF (3 x 3 mL), isopropyl alcohol (3 x 3 mL) and DCM (3 x 3 mL). A second amino acid, *N*-α-Fmoc-L-Phenylalanine (**IXb**, 354 mg, 2.5 eq.), was coupled under the same conditions as described above. The resin was drained and washed with DMF (3 x 3 mL), isopropyl alcohol (3 x 3 mL) and DCM (3 x 3 mL). Upon removal of the Fmoc group with 4 mL of 20% piperidine in DMF (30 min), the resin was washed with DMF (3 x 3 mL), isopropyl alcohol (3 x 3 mL) and DCM (3 x 3 mL). Finally, treatment of the resin with a mixture of 60:40:2 TFA/DCM/water released a crude reaction mixture containing the titled compound **II.1,** which was filtered. Solvents from the filtrate were removed by evaporation under reduced pressure followed by lyophilization. The residue obtained was purified by semipreparative RP-HPLC using aqueous acetonitrile gradient (42% acetonitrile → 80% acetonitrile, 35 min) to give 196 mg of the desired product (yield 55%, ≥98% purity).

Following an analogous procedure to that described for example **II.1** but using different aminoacids, the following products were obtained:

| **Example** | **Compound name** | **HRMS (M + H)⁺:** | |
|---|---|---|---|
| | | **Calculated** | **found** |
| **II.1** | 2-(2-Amino-3-phenylpropionylamino)-*N*-{[({carbamoylmethyl-[2-(2,4-dichlorophenyl)ethyl]carbamoyl}methyl)-(3,3-diphenylpropyl)carbamoyl]methyl}-*N*-[2-(2,4-dichlorophenyl)ethyl]-3-methylbutyramide | C₅₁H₅₇Cl₄N₆O₅ 973.3145 | 973,3129 |
| **II.2** | 2-Amino-4-methylpentanoic acid (1-{{[({carbamoylmethyl-[2-(2,4-dichlorophenyl)ethyl]carbamoyl}methyl)-(3,3-diphenylpropyl)carbamoyl]methyl}-[2-(2,4-dichlorophenyl)ethyl]carbamoyl}-2-methylpropyl)amide | C₄₈H₅₉Cl₄N₆O₅ 939.3301 | 939,3318 |

### Compounds of formula I

### I.1: PGA-II.1

To a solution of PGA sodium salt (**XII,** 0.1 g, 0.5 mmol) with a molecular weight (M_{w}) range 17000-43000 Da in water, 0.2 M HCl was added until the pH of the solution was adjusted to 2.0. The precipitate was collected, dialyzed against water, and lyophilized. Then PGA in its protonic form **(XIIa)** was dissolved in dry DMF (7.5 mL) and DIC (3 eq) and HOBt (3 eq) were added and left stirring for 15 min. Then intermediate 11.1 (0.052 mmol (12 mol%) 1 eq) was added and the pH adjusted to 8 with DIPEA (1.5 mL). The reaction was allowed to proceed at room temperature for 36 h. Thin layer chromatography (TLC, silica) showed complete conversion of **II.1** (R_{f}=0.5) to polymer conjugate (R_{f}=0, MeOH:H₂O:AcOH=85:10:5). To stop the reaction, the mixture was poured into CHCl₃. The resulting precipitate was collected and dried in vacuum. The sodium salt of PGA-peptoid conjugate **I.1** was obtained by dissolving the product in 1.0 M NaHCO₃. This aqueous solution was dialysed against distilled water (M_{W}CO 3,500 Da). Lyophilization of the dialysate yielded the product as a white powder. The total peptoid content in the polymer as determined by UV (λ= 281 nm) was in the range of 30-40 % (w/w) (11-12 mol% functionalization), free drug content < 1wt% total drug.

The average molecular weight (Mw), polydispersity (Mw/Mn) and the behavior of **I.1** and control **XII** in solution were analyzed by size exclusion chromatography (SEC). This technique showed **I.1** as a more compact conformational structure with smaller hydrodynamic volume than **XII** (tr = 12.4 min vs. tr = 11.6 min for **I.1** and **XII,** respectively). By SEC Mw was determined as 35000 Da (Mw/Mn = 1.8) and 32000 Da (Mw/Mn= 1.3) were determined for **XII** and **I.1,** respectively, by GPC using PEG standards.

Following these procedure as for **I.1,** the following examples were obtained:

| **Example** | **Compound name** | **SEC^{a}** | |
|---|---|---|---|
| | | **rt** | **Mw** |
| **I.1: PGA-II.1 conjugate** | Poly-L-glutamic acid [2-(2-Amino-3-phenylpropionylamino)-N-{[({carbamoylmethyl-[2-(2,4-dichlorophenyl)ethyl]carbamoyl}methyl)-(3,3-diphenylpropyl)carbamoyl]-methyl}-*N*-[2-(2,4-dichlorophenyl)ethyl]-3-methylbutyramide] conjugate | 12.4 | 32000 |
| **I.2: PGA-II.2 conjugate** | Poly-L-glutamic acid [2-Amino-4-methylpentanoic acid (1-{{[({carbamoylmethyl-[2-(2,4-dichlorophenyl)ethyl]carbamoyl}methyl)-(3,3-diphenylpropyl)carbamoyl]methyl}-[2-(2,4-dichlorophenyl)ethyl]carbamoyl}-2-methylpropyl)amide] conjugate | 12.5 | 31000 |

| | | | |
|---|---|---|---|
| ^{a} Determined by Size Exclusion Chromatography (SEC) | | | |

### Determination of total drug content by UV spectroscopy

A stock solution of **II.1** in MeOH was prepared (1 mg/mL). To obtain a calibration curve, samples were diluted using MeOH to give a concentration range of 0-1 mg/mL. The total drug loading of the conjugates was determined by measuring the optical density at 272 nm and 281 nm in H₂O. PGA in the same concentration range as **I.1** conjugate analyzed (0-5 mg/mL) in H₂O was used as blank. The extinction coefficient found for **II.1** and **11.2** at 281 nm in MeOH at RT was m = 1103 L mol⁻¹ cm⁻¹ and m = 824 L mol⁻¹ cm⁻¹ respectively.

### Determination of free drug content by HPLC

Aqueous solutions of **I.1** (1 mg/mL) were prepared, and an aliquot (100 µL) was added to a polypropylene tube and made up to 1 mL with water. The pH of the samples was adjusted to 8 with ammonium formate buffer (100 µL, 1 M, pH 8.5), and then CHCl₃ (5 mL) was added. Samples were then thoroughly extracted by vortexing (3 x 10 sec). The upper aqueous layer was carefully removed and the solvent was evaporated under N₂. The dry residue was dissolved in 200 µL of HPLC grade acetonitrile. In parallel the same procedure was carried out for the parent compound **II.1** (using 200µL of a 1 mg/mL stock aqueous solution). Addition of 1 mL of acetonitrile to redissolve the product gave a 200 µg/mL stock from which a range of concentrations was prepared (2 to 100 µg/mL). The free amount of drug in the conjugates was determined by HPLC using a Lichrospher® C18 (150 x 3.9 mm) column. Flow rate 1 mL/min using an acetonitrile gradient in aqueous 0.1 % TFA (from 10 to 90% of acetonitrile in 25 min). The retention time was tr = 22.5 min for **II.1** (λ = 220 nm) and tr = 22.0 for **II.2.**

### Degradation study of XII and II.1/II.2/diglycil on incubation with Cathepsin B. Evaluation of Mw loss and drug release profile from PGA-conaugate.

Cathepsin B (5 U) was added last to a solution of 3 mg PGA or PGA-conjugate **I** in 1 mL of a pH 6 buffer composed by 20 mM sodium acetate, 2 mM EDTA and 5 mM DTT. Incubation was carried out at 37°C. Aliquots (150 µL) were taken at times up to 72 h, immediately frozen in liquid nitrogen, and stored frozen in the dark until assayed by HPLC (analysis of 100 µL aliquots after extraction procedure) and/or GPC (direct analysis of 50 µL aliquots). In control experiments polymers were incubated in buffer alone (without addition of cathepsin B) to assess non-enzymatic hydrolytic cleavage. In addition, free drug (0.75 mg/mL) was also incubated under same conditions and later used as the reference control.
*HPLC Evaluation of Released Drug (VIIIa, I.1 or I.2)* The extraction method described above to determine free drug content of the conjugate samples was also used here. Mass analysis of the released compounds was carried out by MALDI TOF TOF.
*GPC Evaluation of Polymer M_{w} loss.* The 50 µL aliquots were diluted up to 200 µL with buffer (PBS) and the M_{w} determined by GPC using PEG standards. Generally degradation profiles for biomedical polymers are obtained under constant sink conditions and the results correlated with mass loss of the polymer. Since PGA polymers are water soluble, the relative peak areas used in the GPC derived molecular weight calculations provided a suitable alternative to indicate the relative amount of the polymer that remained at each timepoint. As the polymer degraded there was a notable loss of I (PGA-conjugate) molecular weight. Therefore, the loss with time of the area under the curve (AUC) for I and PGA **(XII)** was considered to evaluate the rate of backbone degradation.
To assess degradation in plasma, the same procedure above described was followed using as buffer PBS at pH 7.4 with 10% of FCS.

| **Example** | **Compound name** | **50% Mw Loss^{a} t (h)** | **50% drug^{b} release t (h)** |
|---|---|---|---|
| **I.1:PGA-II.1 conjugate** | Poly-L-glutamic acid [2-(2-Amino-3-phenylpropionylamino)-*N*-{[({carbamoylmethyl-[2-(2,4-dichlorophenyl)ethyl]carbamoyl}methyl)-(3,3-diphenylpropyl)carbamoyl]-methyl}-*N*-[2-(2,4-dichlorophenyl)ethyl]-3-methylbutyramide] conjugate | **5** | **0.7** |
| **I.2: PGA-II.2 conjugate** | Poly-L-glutamic acid [2-Amino-4-methylpentanoic acid (1-{([({carbamoylmethyl-[2-(2,4-dichlorophenyl)ethyl]carbamoyl}methyl)-(3,3-diphenylpropyl)carbamoyl]methyl)-[2-(2,4-dichlorophenyl)ethyl]carbamoyl}-2-methylpropyl)amide] conjugate | **30** | **20** |
| **Diglycil: PGA-GG-VIII conjugate** | Poly-L-glutamic acid [2-Amino-pentanoic acid (1-{{[({carbamoyl[2-(2,4-chlorophenyl)ethyl]carbamoyl}methyl)-(3,3-diphenylpropyl)carbamoyl]methyl}-[2-(2,4-dichlorophenyl)ethyl]carbamoyl}-2-methylpropyl)amide] conjugate | **60^{c}** | **30^{c}** |

| | | | |
|---|---|---|---|
| ^{a} Determined by Size Exclusion Chromatography (SEC) ^{b} Determined by HPLC ^{c}Reference. Vicent MJ and Pérez-Payá, J. Med. Chem. 2006, 49, 3763-3765 | | | |

## Claims

1. A polymer conjugate compound of general formula **I,** 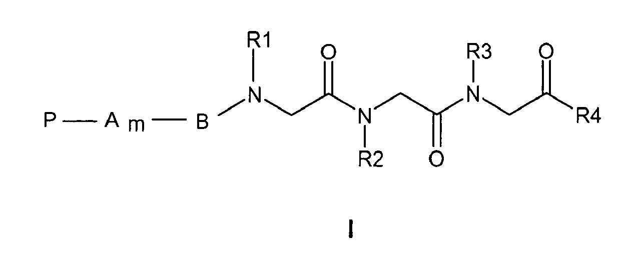 wherein:
R1, R2 and R3 independently represent -(CH₂)₀₋₃-(3-6)Cy, -(CH₂)₀₋₃-(5-6)Hetcy, -(CH₂)₁₋₃-phenyl, -(CH₂)₁₋₃-(1-naphthyl), -(CH₂)₁₋₃-(2-naphthyl), - (CH₂)₁₋₃-(5-10)Hetar, -(CH₂)₂-CH(phenyl)₂, -(CH₂)₂-CH(phenyl)[(5-6)Hetar] or -(CH₂)₂-CH[(5-6)Hetar]₂;
R4 represents -OR5 or -NR5R6
R5 and R6 independently represent -H or -(C₁-C₃)alkyl
(3-6)Cy represents a radical of a 3- to 6-membered partially unsaturated or saturated carbocyclic ring;
(5-6)Hetcy represents a C- or N- radical of a 5- or 6-membered, partially unsaturated or saturated carbocyclic ring containing from one to two heteroatoms independently selected from O, S and N;
Cy and Hetcy can be optionally substituted with one or more substituents selected from halogen, -CF₃ and -OH;
(5-6)Hetar and (5-10)Hetar represent a C- or N- radical of an aromatic 5-or 6-membered and 5- to 10-membered ring respectively; containing from one to four heteroatoms independently selected from O, S and N;
phenyl, 1-naphthyl, 2-naphthyl, (5-6)Hetar and (5-10)Hetar may be optionally substituted with one or more substituents selected from halogen, -CF₃, -OH, -O-(C₁-C₃)alkyl, -CO-(C₁-C₃)alkyl, -(C₁-C₅)alkyl-NR5R6, -NR5R6 and -SO₂NH₂;
B represents an amino acid selected from Val, Leu, Ile, Ala, Arg and Phe;
A independently represents any of the 20 naturally occurring amino acids, and m is 1, 2 or 3, and
P represents a linear or branched polymer selected from polyglutamic acid, polyethylene glycol, polylactic acid, polyglycolic acid, polylactic-coglycolic acid and glycopolymers,
its stereoisomers and mixtures thereof, its polymorphs and mixtures thereof and the pharmaceutically acceptable solvates and addition salts thereof.

2. A compound according to claim 1 wherein R1 represents -(CH₂)₂-phenyl optionally substituted with one or more substituents selected from halogen.

3. A compound according to claim 2 wherein R1 represents 2,4-dichlorophenylethyl.

4. A compound according to claim 1 wherein R2 represents -(CH₂)₁₋₃-aryl optionally substituted, or -(CH₂)₂-CH(Ph)₂ optionally substituted.

5. A compound according to claim 6 wherein R2 represents 3,3-diphenylpropyl.

6. A compound according to claim 1 wherein R3 represents -(CH₂)₂-phenyl optionally substituted with one or more substituents selected from halogen.

7. A compound according to claim 4 wherein R3 represents 2,4-dichlorophenylethyl.

8. A compound according to claim 1 wherein R2 represents 3,3-diphenylpropyl and R1 and R3 both represent 2,4-dichlorophenylethyl.

9. A compound according to claim 1 wherein R4 represents -NR5R6 and R5 and R6 both represent -H.

10. A compound according to claim 1 wherein B represents Val.

11. A compound according to claim 1 wherein B represents Leu.

12. A compound according to claim 1 wherein B represents IIe.

13. A compound according to claim 1 wherein B represents Ala.

14. A compound according to claim 1 wherein B represents Arg.

15. A compound according to claim 1 wherein B represents Phe.

16. A compound according to claim 1 wherein the polyglutamic acid is poly(L-glutamic acid), poly(D-glutamic acid) or poly(DL-glutamic acid).

17. A compound according to claim 16 wherein the polyglutamic acid is poly(L-glutamic acid).

18. A compound according to claim 1 wherein R1 and R3 both represent 2,4-dichlorophenylethyl, R2 represents 3,3-diphenylpropyl, R4 represents - NR5R6, R5 and R6 both represent -H, B represents Val and P represents polyglutamic acid.

19. Use of a compound of formula I as defined in any of claims 1 to 18, or a pharmaceutically acceptable salt or solvate thereof, for the manufacture of a medicament for the treatment or prevention of a condition associated with the induction of apoptosis.

20. Use according to claim 19 wherein the condition is selected from stroke, traumatisms, brain injury, spinal cord injury, meningitis, Alzheimer's disease, Parkinson's disease, Huntington's disease, Kennedy's disease, multiple sclerosis, prion-related Diseases, miocardial ischaemia, as well as other acute or chronic cardiovascular diseases, organ transplantation, alcoholic related pathologies or treatment of allopecia.

21. A method for the treatment or prevention of a condition associated with the induction of apoptosis, said method comprising administering an effective amount of a compound of formula I as defined in any of claims 1 to 18, or a pharmaceutically acceptable salt or solvate thereof.

22. A method according to claim 21 wherein the disease or condition is selected from stroke, traumatisms, brain injury, spinal cord injury, meningitis, Alzheimer's disease, Parkinson's disease, Huntington's disease, Kennedy's disease, multiple sclerosis, prion-related diseases, miocardial ischaemia, as well as other acute or chronic cardiovascular diseases, organ transplantation, alcoholic related pathologies or treatment of allopecia.

23. A pharmaceutical composition comprising a compound of formula I as defined in any of claims 1 to 18, or a pharmaceutically acceptable salt or solvate thereof, and one or more pharmaceutical acceptable excipients.

24. A pharmaceutical composition according to claim 23 in the form of nanospheres, microparticles and nanoparticles.

25. A compound according to any of claims 1 to 18, wherein the ratio of bioactive agent plus peptidic linker to polymer is 25-30% to 75-70% by weight.
